# EUROPÄISCHE PATENTANMELDUNG

(11) **EP 1 102 193 A1**
(43) Veröffentlichungstag der Anmeldung: **23.05.2001**
(21) Anmeldenummer: 00124753.5
(22) Anmeldetag: 13.11.2000
(51) Int. Cl.: G06F 19/00

(54) **Medizinisches System zur Überweisung eines Patienten**

(30) Priorität: 17.11.1999 DE 19955472
(71) Anmelder: SIEMENS AKTIENGESELLSCHAFT, 80333 München (DE)
(72) Erfinder: Schmidt, Volker, Dr., 91054 Erlangen (DE); Schneider, Siegfried, Dr., 91056 Erlangen (DE); Schüll, Hans, 90762 Fürth (DE); Striebel, Werner, 91207 Lauf (DE)

(57) **Zusammenfassung**

Die Erfindung betrifft ein medizinisches System zur Überweisung eines Patienten an eine andere medizinische Dienststelle mit einer Eingabevorrichtung (6, 7, 11) für Überweisungsdaten und qualitätsrelevante Daten, einer Vorrichtung (2, 9, 13) zur Übertragung der Überweisungsdaten an eine Systemzentrale (1), mit einer Vorrichtung (7, 11) zur Abfrage der Überweisungsdaten und mit einer Ausgabevorrichtung (7, 10, 11, 22 bis 24) der Überweisungsdaten, wobei die Systemzentrale (1) eine Vorrichtung (19) zur Erzeugung einer Transaktionsnummer (TN), eine Speichervorrichtung (20) für die Überweisungsdaten sowie qualitätsrelevanter Daten, eine zentrale Datenbank (18) für Struktur-, Prozess-, Ergebnisqualität und/oder Leitlinien und eine Auswertevorrichtung (13) für die Bewertung der qualitätsrelevanten Daten mit den Daten der zentralen Datenbank aufweist.

## Beschreibung

Die Erfindung betrifft ein medizinisches System zur Überweisung eines Patienten an eine andere medizinische Dienststelle mit einer Eingabevorrichtung für Überweisungsdaten, einer Vorrichtung zur Übertragung der Überweisungsdaten an eine Systemzentrale, und mit einer Vorrichtung zur Abfrage der Überweisungsdaten, mit einer Ausgabevorrichtung der Überweisungsdaten.

Die Zusammenarbeit mehrerer Stellen in der Medizin und Gesundheitswesen basiert auf Überweisungsvorgängen. Der Patient wird beispielsweise von einem niedergelassenen Arzt zu einem Kollegen für eine von diesem zu erbringende Diagnostik oder Therapie überwiesen. Gleiches gilt für Krankenhauseinweisungen, für Untersuchungen und Behandlungen innerhalb eines Krankenhauses, für die "Uberweisungs" eines ambulant pflegebedürftigen Patienten an eine ambulante Pflegeeinrichtung, oder die "Uberweisung" eines Patienten mit einem Rezept an eine Apotheke. Alle diese Überweisungsvorgänge zeichnen sich dadurch aus, dass die veranlassende Stelle dafür sorgt, dass bei der durchführenden Stelle für die dort zu leistende Arbeit erforderliche Informationen zusammen mit dem Patienten vorhanden sind. Die ausführende Stelle wiederum sorgt dafür, dass der Überweiser über die Ergebnisse der Überweisung informiert wird. Probleme entstehen bei der Informationsübermittlung, aus der Terminabsprache für die Durchführung angeforderter Maßnahmen, sowie bei der Datenerfassung für die Optimierung der Arbeitsabläufe.

Bei der Informationsübermittlung wird häufig der Patient als Träger der Information eingesetzt, d.h. er trägt die Informationen beispielsweise als Überweisungsschein zur Stelle, die die Überweisung durchführt. Als Folge liegen die Daten erst dann bei der durchführenden Stelle vor, wenn der Patient zum Überweisungstermin erscheint. Damit ist eine Vorplanung der Ressourcen und eine Vorbereitung auf den Patienten, beispielsweise eine OP Planung oder Bestellung der Medikamente, so dass sie beim Eintreffen des Patienten schon da sind, der durchführenden Stelle nicht möglich. Die Menge an Informationen, die auf einem Überweisungsschein mitgegeben werden können, ist häufig nicht ausreichend. Moderne Befundungsverfahren, die beispielsweise zu multimedialen Befunden - Bilder mit Sprache und Annotation - führen, werden heute dem Patienten nicht mitgegeben. Die Rückübermittlung der Informationen der durchführenden Stelle an den Überweiser erfolgt häufig wieder über den Patienten, dabei gehen Informationen verloren, sind erst dann beim Überweiser, wenn der Patient ebenfalls dort ist, oder treffen beispielsweise beim Postversand zu einem späteren Zeitpunkt ein. Damit entfällt die Möglichkeit der Vorbereitung.

Der Überweisungsprozess als Schlüsselprozess im Gesundheitswesen ist prinzipiell für die Erfassung von Qualitätsmerkmalen hinsichtlich der Struktur-, Prozess- und Ergebnisqualität geeignet. Im heutigen, durch Medienbrüche gekennzeichneten System ist die Zusammenführung der im Überweisungsvorgang entstehenden Daten für ein Qualitätsmanagement unmöglich, viele wichtige Daten werden nicht erfasst. So hat beispielsweise ein Arzt keine Möglichkeit, den aktuellen Status seiner Überweisungsvorgänge abzufragen, welche demnächst anstehen, welche bereits durchgeführt werden und welche beendet sind. Überweisungsprozesszeiten zwischen Arztpraxen und zwischen Arztpraxen und Kliniken sowie zwischen Stationen im Krankenhaus werden üblicherweise nicht erfasst. Kodierungen erfolgen typischerweise per Hand oder über einfache Auswahl durch den Anwender am PC.

In der EP 0 905 637 A1 ist eine medizinische Systemarchitektur beschrieben, die einer Vorrichtung zur Erfassung von medizinischen Daten, eine über ein Datennetz zur Übertragung der erfassten Daten mit der Vorrichtung verbundene, externe Speichervorrichtung zur Abspeicherung der erfassten Daten und eine über ein Datennetz mit der Speichervorrichtung verbundenen Befundungsstation zur Auswertung der erfassten Daten aufweist, wobei die Daten der Auswertung in der Speichervorrichtung abgespeichert werden, so dass sie von einer Ausgabestation über ein Datennetz abrufbar sind.

Eine weitere Entwicklung in der Medizin ist die zunehmende Orientierung an Leitlinien. Dabei besteht ein zunehmender Bedarf, die Leitlinien zu evaluieren, d.h. es muss erkannt werden, welche Leitlinien sich bewähren und bei welchen Leitlinien Verbesserungspotentiale bestehen. Die Evaluation erfolgt heute gar nicht oder in medizinischen Studien ähnlich einer Phase 4 Studie bei Medikamenten. Eine kontinuierliche Messung der Qualität einer Leitlinie im laufenden Medizinbetrieb existiert nicht.

Aus der US 5,583,758 ist ein medizinisches Management System bekannt, das als Qualitätsmaß für Untersuchungen den Abstand der Realität zu vorgegebenen Richtlinien misst.

Die Erfindung geht von der Aufgabe aus, ein System der eingangs genannten Art derart auszubilden, dass eine automatische, computergestützte Kodierung medizinischer Sachverhalte für die Bewertung des Überweisungsvorganges, eine automatische Erfassung von Indikatoren für die Struktur- und Prozessqualität des Überweisungsprozesses sowie ein laufendes Monitoring eingesetzter Leitlinien und ihrer Ergebnisse ermöglicht wird. Gleichzeitig wird die freie Arztwahl und die medizinische Schweigepflicht berücksichtigt.

Die Aufgabe wird erfindungsgemäß dadurch gelöst, dass die Systemzentrale eine Vorrichtung zur Erzeugung einer Transaktionsnummer, eine Speichervorrichtung für die Überweisungsdaten sowie qualitätsrelevanter Daten, eine zentrale Datenbank für Struktur-, Prozess-, Ergebnisqualität und/oder Leitlinien und eine Auswertevorrichtung für die Bewertung der qualitätsrelevanten Daten mit den Daten der zentralen Datenbank aufweist. Für jede Überweisung wird von der Systemzentrale eine eindeutige Transaktionsnummer erzeugt, die dem Patienten auf einem Papier-Überweisungsschein mitgegeben werden kann oder die vom überweisenden Arzt an die durchführende Stelle über andere Medien (E-Mail, Fax, Telefon, oder andere) übermittelt werden kann, so dass die freie Arztwahl für den Patienten erhalten bleibt. Mit Kenntnis dieser Transaktionsnummer können im einfachsten Fall bei der durchführenden Stelle die vom Überweiser in das System eingestellten Daten abgerufen werden. Dabei wird der gesamte Bereich, den der Patient durchlaufen kann, wie Hausarzt - Facharzt - Klinik abgedeckt.

Durch dieses medizinische System lässt sich der Überweisungsvorgang als Werkzeug für ein Qualitätsmanagement, beispielsweise zur Messung der Nutzung von Ressourcen im Gesundheitssystem verwenden. Auch lässt er sich zur automatisierten, kontinuierlichen Evaluation von Richtlinien und Richtlinienteilen einsetzen. Der Überweisungsprozess wird dadurch messbar bezüglich Struktur-, Prozess- und Ergebnisqualität.

Es hat sich als vorteilhaft erwiesen, wenn das System eine Kontrollvorrichtung zur Kontrolle anhand von Leitlinien oder Qualitätsmerkmalen aufweist, ob alle erforderlichen Maßnahmen oder Informationen erfolgt sind. Dadurch lässt sich der Outcome messen, d.h. die Größen, die unter dem Einfluss einer Richtlinie stehen wie beispielsweise die Radiologie-Ausnutzung oder wie häufig wird eine Untersuchung durchgeführt. Es kann damit der Bedarf und die Qualität einer Richtlinie überprüft werden. Die Richtlinie kann im laufenden Betrieb verbessert werden.

Eine einfache Zuordnung der Ergebnisse zu den Patientendaten kann erfolgen, wenn das System eine Eingabevorrichtung für das Ergebnis der Überweisung aufweist, das aufgrund der Transaktionsnummer mit den Daten der Anforderung der Überweisung in der Speichervorrichtung abspeicherbar ist.

In vorteilhafter Weise kann die Systemzentrale eine Speichervorrichtung für elektronische Patientenakten aufweisen, an der die Überweisung ankoppelbar ist. Dadurch können Überweisungs-relevante Daten übernommen und Ergebnisdaten in der Patientenakte abgespeichert werden.

Eine Auswertung für das Qualitätsmanagement für die medizinische Ergebnisqualität wird ermöglicht, wenn die Eingabevorrichtung derart ausgebildet ist, dass sie eine Kodierung medizinischer Sachverhalte aus dem eingegebenen Text heraus bewirkt. Es werden der medizinische Grund der Überweisung und das medizinische Ergebnis der Überweisung kodiert. Dazu werden die in medizinischer Terminologie beschriebenen Sachverhalte auf existierende oder sich entwickelnde, ggf. internationale Standards abgebildet, beispielsweise ICD9, ICD10, ICPM oder andere. Die Kodierung kann automatisch erfolgen oder anhand von Auswahltabellen, die dem Anwender präsentiert werden. Durch die automatische Kodierung aus dem eingegebenen Text werden diese Texteingaben besser auswertbar als der alleinige Text.

Bei einem Menüsystem hangelt man sich ausgehend von gewünschter Fachrichtung durch einen Menübaum durch, bis man zu den gewünschten Daten kommt. Eine solche Kette könnte sein: Krankenhaus - Chirurgie - akutes Abdomen - verdacht auf akute Appendizitis - zur Appendektomie.

Bei lexikalischen Suchsysteme gibt der Anwender die Anfangsbuchstaben der Fragestellung/Untersuchung ein, und wählt dann aus der Liste aus.

Bei einem information retrieval System schreibt der Anwender im Klartext, wie er es bisher auf den Überweisungen auch gemacht hat. Über ein Textanalysesystem wird in der Anmeldung nach Schlüsselworten gesucht, dem Anwender wird dann die Kodierung samt Beschreibung vorgeschlagen.

Die Überweisungen lassen sich beispielsweise über eine systeminterne Patienten-ID pseudonymisieren, wenn die Eingabevorrichtung derart ausgebildet ist, dass eine Verschlüsselung der patientenspezischen Daten erfolgt. Nicht addressierte Vertraulichkeit kann durch die Verschlüsselung realisiert werden. Eine zusätzliche Verschlüsselung der medizinischen Daten kann beim Überweiser in der Art erfolgen, dass die medizinischen Daten im zentralen System mit Standardtechnologien verschlüsselt abgelegt werden und nur von der durchführenden Stelle entschlüsselt werden können.

Die Möglichkeit einer statistischen Auswertung von nicht geheimen Daten durch jedermann kann erfolgen, wenn die Eingabevorrichtung derart ausgebildet ist, dass nur ein Teil der patientenspezischen Daten verschlüsselt werden, wobei die Transaktionsnummer verknüpft mit einer Verschlüsselung mit mehrstufigem Verfahren ist. Lediglich der verschlüsselte Teil kann nur vom Schlüsselinhaber, dem Arzt oder einer Arztgruppe, entschlüsselt und ausgewertet werden.

Es hat sich als vorteilhaft erwiesen, wenn die Eingabevorrichtung für Überweisungsdaten ein wissensbasiertes System aufweist, das mit einem Speicher für relevante Leitlinien zur krankheitsspezifischen Führung des Überweisers bei der Eingabe verbunden ist.

Informationen bezüglich der Kosten, beispielsweise für Untersuchungen, Transporte oder Arztbesuche erhält man, wenn die Systemzentrale ein Kostenmodul für die im Überweisungsprozess anfallenden Kosten aufweist und dass die Auswertevorrichtung zur Durchführung von Kosten-Nutzen Analysen ausgebildet ist.

Eine eindeutige Identifikation des Patienten und die Zuordnung zur Überweisung erhält man, wenn an der Eingabevorrichtung eine digitale Photokamera zur digitalen Erfassung optischer Bilder angeschlossen ist.

Die Erfindung ist nachfolgend anhand eines in der Zeichnung dargestellten Ausführungsbeispieles näher erläutert.

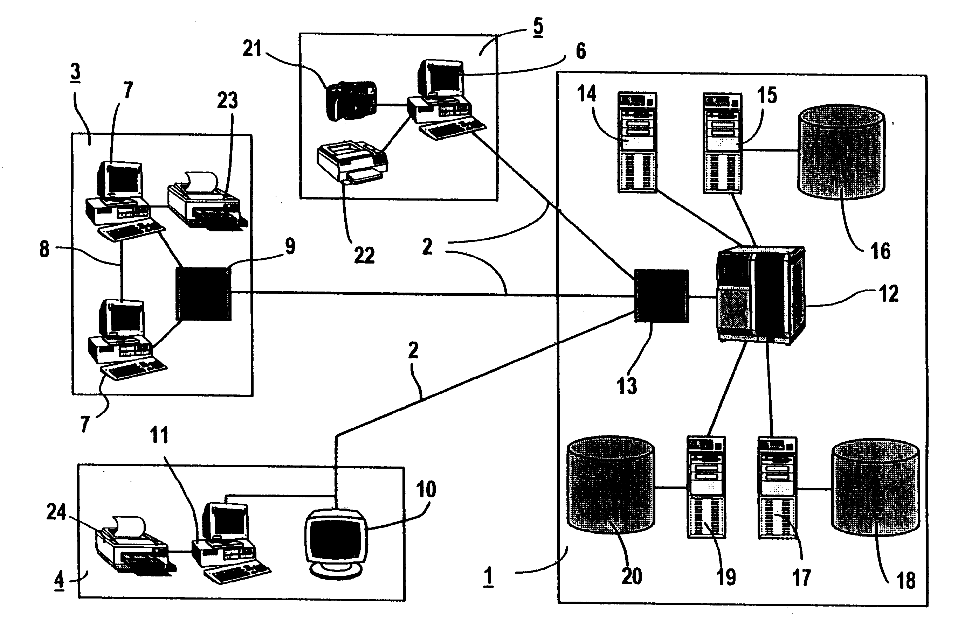

In der Figur 1 ist ein erfindungsgemäßes System zur Überweisung eines Patienten mit einer Systemzentrale 1 dargestellt, an der über ein ISDN-Netz 2 mehrere Krankenhäuser 3, Gemeinschaftspraxen 4 von Ärzten und Einzelpraxen 5 von erstbehandelnden, niedergelassenen Ärzten mit einem Personal Computer (PC) 6 als Eingabevorrichtung angeschlossen sind. Die Krankenhäuser 3 können mehrere Workstations 7 zur Eingabe von Patientendaten und Befundungsergebnissen aufweisen, die an einem Krankenhaus Informationssystem KIS 8 angeschlossen sind, das über ein ISDN-Interface 9 mit dem ISDN-Netz 2 verbunden ist. Die Gemeinschaftspraxen 4 weisen mehrere Sicht- 10 oder Befundungsstationen und Personal Computer (PC) 11 auf.

In der Systemzentrale 1 ist ein Gateway 12 vorgesehen, das an dem ISDN-Netz 2 über ein ISDN-Interface 13 angeschlossen ist. An dem Gateway 12 kann ein Internet-Proxy-Server 14 zum Zugriff auf das Internet, ein Patientendaten-Server 15 zur Verwaltung der in einer Datenbank 16 gespeicherten Patientendaten, eine Auswertevorrichtung 17 für die Bewertung von qualitätsrelevanten Daten mit den Daten einer zentralen Datenbank 18 für Struktur-, Prozess-, Ergebnisqualität und/oder Leitlinien, und mit einer Vorrichtung 19 zur Koordinierung der Überweisungen und der anderen Daten angeschlossen sein, die mit einer Speichervorrichtung 20 für die Überweisungsdaten sowie qualitätsrelevanter Daten verbunden ist. Zur Koordinierung der Überweisungen erzeugt die Vorrichtung 19 eine Transaktionsnummer TN, die der jeweiligen Überweisung fest zugeordnet ist.

Die in den Speichern 16, 18 und 20 abzuspeichernden Daten können durch die vorgeschalteten Server 15, 17 und 19 verschlüsselt werden.

An dem Personal Computer (PC) 6 der Einzelpraxis 5 kann eine digitale Photokamera 21 zur digitalen Erfassung optischer Bilder angeschlossen sein. Weiterhin ist der PC 6 mit einem Drucker 22 verbunden. Sowohl die Krankenhäuser 3 als auch die Gemeinschaftspraxen 4 sind mit Druckern 23 und 24 ausgestattet.

Mit dem erfindungsgemäßen System erfolgt eine elektronische Erstellung eines Begleitbriefes auf dem PC 6 und Vergabe einer Transaktionsnummer TN durch die Vorrichtung 19 bei Anlegen eines Überweisungsformulars, das auf dem Drucker 22 ausgedruckt und dem Patienten übergeben werden kann. Der Patient kann die durchführende Stelle anrufen und die Transaktionsnummer vorab durchgeben. Damit stehen dann erforderliche Daten für die Vorbereitung zur Verfügung. Der durchführende Arzt gibt die TN über die Workstations 7 oder den PC 11 ein und ruft die bisher gespeicherten Informationen von der Systemzentrale 1 ab, dokumentiert sein Ergebnis und ordnet über die TN seine neuen Daten den bisher gespeicherten zu.

Die Ergebnis-Daten werden direkt während des bzw. nach dem Patienten-Besuch beim durchführenden Arzt in der Systemzentrale 1 abgespeichert und stehen daraufhin unmittelbar dem veranlassenden Arzt zur Verfügung. Der veranlassende Arzt erhält jederzeit Informationen über den Status der Überweisung (noch offen, abgeschlossen). Bei terminabhängigen Überweisungen kann für den veranlassenden Arzt und den Patienten eine Erinnerung geschaltet werden.

Wenn an der Eingabevorrichtung die digitale Photokamera 21 angeschlossen ist, können die Photos gemeinsam mit den medizinischen Bildern und patientenbezogenen Daten in der Systemzentrale 1 eingespeichert werden. Diese Photos des Patienten lassen sich zusätzlich zur Adresse auf dem Überweisungsformular wiedergeben.

Auch kann an der Eingabevorrichtung eine automatische Kodierung medizinischer Fragestellungen und Ergebnisse nach ICD, ICPM o.a. erfolgen. Kopien von digital vorliegenden oder eingescannten Dokumenten lassen sich ebenso wie Multimedia Befunde an die elektronische Überweisung anhängen.

Durch die gleichzeitige Speicherung von qualitätsrelevanten Daten wie Prozess-, Struktur- und Ergebnisdaten können diese von der Vorrichtung 19 in der Systemzentrale 1 ausgewertet und von den Teilnehmern an Überweisungen abgefragt werden.

In einem Datensatz können Informationen aus den Bereichen Veranlassende Stelle, Durchführende Stelle, Patient, Kostenträger, Medizinischer Kontext, Ergebnisse, Vorschläge und Zeitpunkte bei einer Aktion, einer Anmeldung, einer Überweisung, einer Durchführung oder einer Befundung, erfasst werden:

Der Patient lässt sich beispielsweise durch ein Eingabesystem im Wartezimmer einbinden, wo der Patient über die Transaktionsnummer seine Sicht über den Prozess in strukturierter Form eingeben und abrufen kann. Er kann auch allgemeiner von einem Kiosksystem oder von einem WEB-TV aus seine Informationen abrufen.

Durch das erfindungsgemäße System wird bei der Informationsübermittlung das Ausfüllen der Überweisungsformulare durch die durchführende Stelle unterstützt. Durch den gleichzeitigen Zugriff auf die Systemzentrale 1 ist eine Rücksendung des Ergebnisberichtes nicht mehr erforderlich. Der veranlassende Arzt hat jederzeit Information über den Stand zusätzlicher Maßnahmen. Die Patienten lassen sich eindeutig identifizieren.

Beim Qualitätsmanagement kann der Nachweis der Qualität von medizinischen Leistungen erfolgen. Texteingaben für Anamnese, Diagnose und Therapie können "standardisiert" und auswertbar gemacht werden. Überweisungsqualität ist messbar bezüglich Struktur-, Prozess- und Ergebnisqualität. Dazu wird ein Datensatz definiert, der sinnvolle Messgrößen erfasst.

Bei den Leitlinien erfolgt eine Evaluation sowie die Überprüfung der Einhaltung von Leitlinien

Weiterer medizinischer Kontext kann erfaßt werden, wenn im System Computergestützte Richtlinien verwendet werden. Richtlinien werden in einer eigenen Datenbank verwaltet und über den zentralen Server zur Verfügung gestellt. Dabei werden für die ambulanten Praxen Kopien versendet, auf den lokalen Rechnern laufen Shell-Systeme, in denen die Richtlinien ausgeführt werden können. Das kann so aussehen, dass in einer durch die Richtlinie gesteuerte Befragung die Richtliniendaten eingibt, oder dass über eine Schnittstelle zum Praxissystem zum Krankenhausinformationssystem die Daten automatisch eingelesen werden.

Jede Richtlinie hat eine im Netzwerk eindeutige Kennung. Wenn eine Richtlinie verwendet wurde, dann wird die Richtlinienkennung in der Überweisung eingetragen und im zentralen Server in der Datenbank zusammen mit den übrigen Daten abgelegt. Auch der Richtlinienpfad wird kodiert und abgespeichert.

In jedem Falle sind als rechtliche Randbedingungen die Speicherung der Daten der ausführenden Stelle in Beziehung zur veranlassenden Stelle bei freier Arztwahl, Apothekenwahl, Krankenhauswahl, Pflegedienstwahl des Patienten zuzulassen, die medizinische Schweigepflicht und den Datenschutz zu gewährleisten sowie die Dokumentationspflicht zu befolgen.

Bei dem erfindungsgemäßen EDV System werden die im Überweisungsvorgang anfallenden Daten über eine Systemzentrale 1 berechtigten Stellen zur Verfügung gestellt. Der Überweisungsvorgang umfasst folgende Schritte:
- Dateneingabe am PC (Überweiser) oder an einem technisch geeigneten Eingabegerät
- Zusätzlich die Möglichkeit, die Qualität der Dateneingabe im laufenden Prozess zu kontrollieren, - z.B. anhand von Leitlinien oder Qualitäts-Dokumentationsbögen. Kontrolle, ob alle erforderlichen Maßnahmen / Informationen erfolgt sind.
- Zusätzlich die Möglichkeit, die Dateneingabe an wissensbasierte Systeme zu koppeln, die z.B. krankheitsspezifisch den Überweiser führen. Die wissensbasierten Systeme holen vom zentralen Server oder aus dem Internet die für den Fall relevanten Leitlinien und präsentieren sie dem Anwender.
- Generierung einer Transaktionsnummer am zentralen Server.
- Ausdruck eines Formulars an den Patienten.
- Ggf. Automatische Kodierung medizinischer Sachverhalte bei der Eingabe.
- Ggf. Verschlüsselung der patientenspezischen Daten am Client, d.h. zusätzlich Möglichkeit, die Überweisung zu pseudonymisieren, z.B. über eine systeminterne Patienten-ID. Nicht addressierte Vertraulichkeit kann durch die Verschlüsselung realisiert werden.
- Ggf. elektronische Signatur der Daten (HCP-konform).
- Datenübermittlung an zentralen Server.
- Speicherung qualitätsrelevanter Daten sowie der eigentlichen überweisungsspezifischen Daten.
- Abruf der Daten, sobald Patient Transaktionsnummer an durchführende Stelle durchgegeben hat.
- Nutzung der Transaktionsnummer, um Anforderung und Ergebnis zusammenzuführen.
- Zusätzliche Möglichkeit die Überweisung an eine zentrale elektronische Patientenakte anzukoppeln - Übernahme von Überweisungs-relevanten Daten, Speicherung von Ergebnisdaten in der Akte
- Möglichkeit, die zentrale QM - Datenbank auszuwerten
- Zusätzlich die Möglichkeit, das System an ein Workflow System zu koppeln
- Zusätzlich die Möglichkeit, das System an ein Kostenmodul anzukoppeln, dieses liefert Informationen bezüglich der im Überweisungsprozess anfallenden Kosten, z.B. für Untersuchungen, Transporte, Arztkosten, ... Bei dieser Ankopplung können die Qualitäts-Daten für Kosten-Nutzen Analysen verwendet werden.
- Zusätzliche Möglichkeit, Patientenbefragungen bezüglich der wahrgenommenen Qualität des Überweisungsprozess einzubinden.

Für die eine Überweisung anfordernde Stelle ergeben sich der Überblick und Nachverfolgbarkeit von angestoßenen Überweisungen sowie die Unterstützung bei der Kodierung medizinischer Sachverhalte als Vorteile.

Vorteilhaft für die durchführende Stelle ist die einfache Zuordnung von Anfrage zu Ergebnis sowie die sofortige Erreichbarkeit der Ergebnisse durch die anfordernde Stelle.

Für das Gesundheitssystem ergeben sich folgende Vorteile:
- Überweisungen können automatisch bezüglich veranlassender Stelle, durchführender Stelle, medizinischer Fragestellung und medizinischem Ergebnis ausgewertet werden. Damit fallen Daten für das Qualitätsmanagement an, die für Ressourcenplanung sowie zur Identifikation schlechter und guter Nutzung verwendet werden können.
- Automatische Kodierung kann mit messbarer, definierter Fehlerhäufigkeit arbeiten. Die Aussagen sind verlässlicher als bei Codierung von Hand.
- Leitlinien können im laufenden Betrieb überwacht werden, sowohl als ganze Leitlinie, als auch in Zusammenhang mit dem patientenspezifischen Kontext.

## Patentansprüche

1. Medizinisches System zur Überweisung eines Patienten an eine andere medizinische Dienststelle mit einer Eingabevorrichtung (6, 7, 11) für Überweisungsdaten und qualitätsrelevante Daten, einer Vorrichtung (2, 9, 13) zur Übertragung der Überweisungsdaten an eine Systemzentrale (1), mit einer Vorrichtung (7, 11) zur Abfrage der Überweisungsdaten und mit einer Ausgabevorrichtung (7, 10, 11, 22 bis 24) der Überweisungsdaten, wobei die Systemzentrale (1) eine Vorrichtung (19) zur Erzeugung einer Transaktionsnummer (TN), eine Speichervorrichtung (20) für die Überweisungsdaten sowie qualitätsrelevanter Daten, eine zentrale Datenbank (18) für Struktur-, Prozess-, Ergebnisqualität und/oder Leitlinien und eine Auswertevorrichtung (13) für die Bewertung der qualitätsrelevanten Daten mit den Daten der zentralen Datenbank aufweist.

2. Medizinisches System nach Anspruch 1, **dadurch gekennzeichnet,** dass das System eine Kontrollvorrichtung (17) zur Kontrolle anhand von Leitlinien oder Qualitätsmerkmalen aufweist, ob alle erforderlichen Maßnahmen oder Informationen erfolgt sind.

3. Medizinisches System nach Anspruch 1 oder 2, **dadurchgekennzeichnet,** dass das System eine Eingabevorrichtung (7, 11) für das Ergebnis der Überweisung aufweist, das aufgrund der Transaktionsnummer (TN) mit den Daten der Anforderung der Überweisung in der Speichervorrichtung (20) abspeicherbar ist.

4. Medizinisches System nach einem der Ansprüche 1 bis 3, **dadurch gekennzeichnet**, dass die Systemzentrale (1) eine Speichervorrichtung für elektronische Patientenakten (16) aufweist, an der die Überweisung ankoppelbar ist.

5. Medizinisches System nach einem der Ansprüche 1 bis 4, **dadurch gekennzeichnet**, dass die Eingabevorrichtung (6, 7, 11) derart ausgebildet ist, dass sie eine Kodierung medizinischer Sachverhalte aus dem eingegebenen Text heraus bewirkt.

6. Medizinisches System nach einem der Ansprüche 1 bis 5, **dadurch gekennzeichnet**, dass die Eingabevorrichtung (6, 7, 11) derart ausgebildet ist, dass eine Verschlüsselung der patientenspezischen Daten erfolgt.

7. Medizinisches System nach Anspruch 6, **dadurch gekennzeichnet**, dass die Eingabevorrichtung (6, 7, 11) derart ausgebildet ist, dass nur ein Teil der patientenspezischen Daten verschlüsselt werden, wobei die Transaktionsnummer verknüpft mit einer Verschlüsselung mit mehrstufigem Verfahren ist.

8. Medizinisches System nach einem der Ansprüche 1 bis 7, **dadurch gekennzeichnet**, dass die Eingabevorrichtung (6) für Überweisungsdaten ein wissensbasiertes System aufweist, das mit einem Speicher für relevante Leitlinien zur krankheitsspezifischen Führung des Überweisers bei der Eingabe verbunden ist.

9. Medizinisches System nach einem der Ansprüche 1 bis 8, **dadurch gekennzeichnet**, dass die Systemzentrale (1) ein Kostenmodul für die im Überweisungsprozess anfallenden Kosten aufweist und dass die Auswertevorrichtung zur Durchführung von Kosten-Nutzen Analysen ausgebildet ist.

10. Medizinisches System nach einem der Ansprüche 1 bis 9, **dadurch gekennzeichnet**, dass an der Eingabevorrichtung (6) eine digitale Photokamera 20 zur digitalen Erfassung optischer Bilder angeschlossen ist.
